# EUROPEAN PATENT APPLICATION

(11) **EP 2 439 187 A1**
(43) Date of publication of application: **11.04.2012**
(21) Application number: 11189860.7
(22) Date of filing: 19.12.2008
(51) Int. Cl.: C07C 67/03, C10M 105/38, C07H 13/06

(54) **Alkoxylated carbohydrate esters and their use**

(62) Divisional of application: 08022118.7
(71) Applicant: Cognis IP Management GmbH, 40589 Düsseldorf (DE)
(72) Inventor: Mainx, Hans-Georg, 42799 Leichlingen (DE); Hofer, Peter, 40589 Düsseldorf (DE); Busch, Stefan, 40597 Düsseldorf (DE); Mahnke, Eike-Ulf, 42553 Velbert (DE)
(74) Representative: Fabry, Bernd

(57) **Abstract**

Suggested are new adducts of on average 1 to 80 moles ethylene oxide and/or propylene oxide to esters of carbohydrates selected from the group consisting of dextrose, saccharose, maltose and maltotriose.

## Description

### Field of the invention

The present invention is related to the area of polyol esters and refers to specific alkoxylated carbohydrate esters with improved colour and odour quality obtained by use of specific transesterification catalysts, and several applications of the new products.

### Background of the invention

Polyol esters, like for example fatty acid esters of pentaerythritol, are well known products of organic chemistry which are employed in various areas of application, as for example lubricants or cosmetic thickeners. It is common to obtain these products from transesterification of polyols and fatty acid esters, in particular fatty acid methyl esters or glycerides. Typically, alkaline catalysts, in particular alkaline or alkaline earth oxides, hydroxides or methylates [US 4,942,228 (P&G)] as well as metal organic compounds like e.g. dibutyl tin diacetate are employed. In order to achieve a sufficient degree of transesterification it is necessary to conduct the reaction at high temperatures up to 250 °C. As a result, the products thus obtained show dark colours and exhibit undesired odours, what makes an after-treatment using bleaches and reducing agents mandatory. Unfortunately, often even intensive bleaching operation will not provide products of acceptable colour. This is particularly the case for the production of heat-sensitive carbohydrate esters.

It has therefore been the object of the present invention to develop new carbohydrate esters based on heat-sensitive carbohydrates and showing a high degree of alkoxylation, exhibiting an improved colour and odour quality, are free of unwanted impurities and which are particularly useful as additives for agrochemical applications.

### Detailed description of the invention

The present invention refers to adducts of on average 1 to 80 moles ethylene oxide and/or propylene oxide to esters of carbohydrates selected from the group consisting of dextrose, saccharose, maltose and maltotriose.

Surprisingly it has been observed that these products can be obtained in high yields and good colour and odour quality and free of unwanted impurities, such as in particular heavy metals like for example tin. Said esters are obtainable by transesterification of alkoxylation products of said specific carbohydrates with fatty acid esters. The new products show a quality that allows using them also in areas like cosmetics or nutrition where the presence of heavy metals even in traces is inadmissible.

### Alkoxylated carbohydrate part of the ester molecule

Suitable carbohydrates and respectively adducts of ethylene and/or propylene oxide to these carbohydrates are selected from the group consisting of dextrose, maltose, maltotriose or saccharose. The degree of alkoxylation can vary between 1 and 80. Particularly suitable starting materials are adducts of on average about 1 to about 50, preferably about 5 to about 40 and more preferably about 10 to about 30 moles ethylene oxide and/or propylene oxide to said carbohydrates. The alkoxylation products may show a blockwise or statistical distribution of the alkylene oxide units.

### Fatty acid part of the ester molecule

The carbohydrate esters of the present invention are obtained from transesterification of the alkoxylated carbohydrates with fatty acid esters. Typically said fatty acid esters follow general formula (I), R¹CO-OR² (I)

in which R¹CO represents a linear or branched, saturated or unsaturated, optionally hydroxy-functionalised acyl radical having about 6 to about 30 carbon atoms and 0 or 1 to 3 double bonds, and R² represents an alkyl radical having 1 to about 6 carbon atoms or the residue of glycerol. The fatty acid esters suitable to be used in the inventive process therefore represent either alkyl esters or (mono/di/tri) glycerides.

Typical examples of suitable esters can be chosen from methyl, ethyl, propyl, butyl, pentyl or hexyl esters of capronic acid, caprylic acid, caprinic acid, lauric acid, myrystic acid, palmitic acid, palmoleic acid, stearic acid, isostearic acid, oleic acid, elaidinic acid, linoleic acic, conjugated linoleic acid, linolenic acid, 12-hydroxy stearic acid, ricinoleic acid, gadoleic acid, arachidonic acid, behenic acid, erucic acid and their technical mixtures like for example tallow acid, coco fatty acid, palm fatty acid, sunflower acid, or soy acid. Instead of the alkyl esters mono-, di- and/or triglycerides of the same fatty acids can also be used for transesterification. Finally, it is also possible to use natural fats and oils, for example sunflower or soy oil for the same purpose.

### Transesterification catalysts

The new carbohydrates are obtainable for the first type, since new catalysts have been developed which allow conducting the reaction under conditions that the resulting products show excellent colour and low colour; at the same time the esters are free of unwanted traces of heavy metals. The new catalysts represent reducing mineral or organic acid selected from the group consisting of
(i) sulphuric or sulphonic acids with an oxidation value of sulphur of less than 6 or their salts, and/or
(ii) phosphoric or phosphonic acids with an oxidation value of phosphor of less than 5 or their salts.

Typical examples for reducing mineral or organic are sulphuric or sulphonic acids selected from the group consisting of sulphurous acid, dithionic acid, sulphinic acid and organic sulphinic acids and their alkali or alkaline earth salts. It is also possible to apply mineral or organic acids represent phosphorous or phosphonic acids selected from the group consisting of phosphorous acid, diphosphorous acid, hypophosphorous acid, and hypodiphosphorous acid or their alkaline or alkaline earth salts. Preferred salts are potassium salts.

### Transesterification

Transesterification is conducted in a manner known from organic chemistry. Typically one component - usually the one being less expensive or easier to separate - is used in molar excess. Typically, the catalysts are prepared in-situ: stoichiometrical amounts of mineral acid and alkali hydroxide are added to the ester/polyol-mixture. It has been found advantageous to use the catalysts in concentrations of about 0.05 to about 1 % b.w., preferably about 0.1 to about 0.5 % b.w. calculated on the transesterification starting materials. Once the catalyst has been added, the mixture is heated up to the final temperature either under nitrogen or vacuum, depending on the reactants. Suitable reaction temperatures are in the range of about 120 to about 200 °C, preferably about 150 to about 180 °C. It is preferred to conduct the transesterification under reduced pressure, for example 1 to about 300 mbar and preferably about 10 to about 100 mbar. Different to the catalysts known from the state of the art the process according to the present invention can be conducted under mild conditions, preferably at pH values in between about 6 and about 8.. In particular running the reaction under neutral pH conditions was surprising and has not been expected. The esters obtained by this procedure are light coloured and do not need any bleaching after the reaction.

### Carbohydrate esters

In particular the present invention refers to adducts of on average about 5 to about 60 and preferably about 10 to about 50 moles ethylene oxide and/or propylene oxide to esters of carbohydrates selected from the group consisting of dextrose, saccharose, maltose and maltotriose. A particular preferred product is an adduct of on average 40 moles ethylene oxide and 6 moles propylene oxide to saccharose tetraoleate.

### Industrial application

The new carbohydrate esters cited above are useful for quite a number of different applications. The present invention therefore also covers their use
o for making agrochemical formulations;
o for making blended lubes;
○ for making coated additives;
○ for making paint additives;
○ for making cosmetic and/or pharmaceutical compositions; and
○ as food additives.

### Example 1

### Transesterification in the presence of hypophosphites

475 g (440 g active, 0,36 mol) of an adduct of on average 20 moles propylene oxide to saccharose were mixed with 276 g (1,1 mol) of a technical grade C₁₂-C₁₈ fatty acid methyl ester (Edenor^{®} MeC12-18, BASF Personal care & Nutrition GmbH) and 15 g of a 25 % solution of potassium hypophosphite. The mixture was set under a 300 mbar vacuum and heated up slowly under stirring to 180°C. After the removal of the water the reaction started vigorously at a temperature of about 140 to 150 °C. After the removal of the first large amount of methanol the vacuum was slowly reduced to < 1 mbar and the reaction mixture was kept under these conditions for another 3 to 4 hours. Once the reaction was completed the final ester was cooled to room temperature.

| | |
|---|---|
| Yield: | approx. 670 g ester |
| Appearance: | clear yellowish liquid with a fatty smell |
| pH-value: | 6.2 |

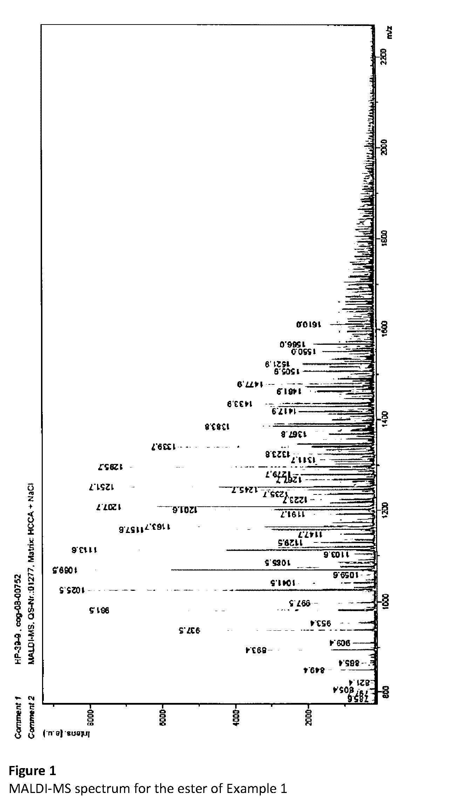

The corresponding MALDI-MS spectrum if ester thus obtained is shown in Figure 1.

### Comparison Examples C1 and C2

### Transesterification in the presence of other catalysts

Inventive example 1 was repeated replacing the hypophosphite catalyst by (a) 0.3 % b.w. potassium hydroxide and (b) 0.3 % b.w. sodium methylate. In both experiments dark brown to black liquids with a smoky smell were obtained

### Example 2

### Transesterification in the presence of hypophosphites

Inventive example 1 was repeated using 85 g trimethylol propane, 565 g of a sunflower fatty acid methyl ester and 15 g of a 25 % b.w. solution of the potassium salt of hypophosphorous acid as the catalyst. One obtained a yield of 620 g ester in the form of clear colourless liquid with a fatty smell.

## Claims

1. Adducts of on average 1 to 80 moles ethylene oxide and/or propylene oxide to esters of carbohydrates selected from the group consisting of dextrose, saccharose, maltose and maltotriose.

2. Carbohydrate esters according to Claim 1, **characterized in that** ester component is derived from fatty acid esters of formula (I)
R¹CO-OR² (I)
in which R¹CO represents a linear or branched, saturated or unsaturated, optionally hydroxy-functionalised acyl radical having 6 to 30 carbon atoms and 0 or 1 to 3 double bonds, and R² represents an alkyl radical having 1 to about 6 carbon atoms or the residue of glycerol.

3. Carbohydrate esters according to Claims 1 and/or 2, **characterized in that** they comprise 1 to 50 moles ethylene oxide and/or propylene oxide.

4. Carbohydrate esters according to Claims 1 and/or 2, **characterized in that** they comprise 5 to 40 moles ethylene oxide and/or propylene oxide.

5. Carbohydrate esters according to Claims 1 and/or 2, **characterized in that** they comprise 10 to 30 moles ethylene oxide and/or propylene oxide.

6. An adduct of on average 40 moles ethylene oxide and 6 moles propylene oxide to saccharose tetraoleate.

7. Use of carbohydrate esters according to Claims 1 and/or 6 for making agrochemical formulations.

8. Use of carbohydrate esters according to Claims 1 and/or 6 for making blended lubes.

9. Use of carbohydrate esters according to Claims 1 and/or 6 for making coated additives.

10. Use of carbohydrate esters according to Claims 1 and/or 6 for making paint additives.

11. Use of carbohydrate esters according to Claims 1 and/or 6 for making cosmetic and/or pharmaceutical compositions.

12. Use of carbohydrate esters according to Claims 1 and/or 6 as food additives.
